# EUROPÄISCHE PATENTANMELDUNG

(11) **EP 2 036 493 A1**
(43) Veröffentlichungstag der Anmeldung: **18.03.2009**
(21) Anmeldenummer: 08161980.1
(22) Anmeldetag: 07.08.2008
(51) Int. Cl.: A61B 5/0408, A61B 5/0488, A61N 1/04, A61B 5/0492

(54) **Größenvariable medizinische Elektrode mit Anfassbereich**

(30) Priorität: 14.09.2007 DE 102007044019
(71) Anmelder: Dräger Medical AG & Co. KG, 23558 Lübeck (DE)
(72) Erfinder: Grassl, Thomas, Dr., 23560 Lübeck (DE)
(74) Vertreter: Strauss, Steffen

(57) **Zusammenfassung**

Die Erfindung betrifft eine medizinische Elektrode zur Erfassung und Übertragung von elektrischen Impulsen von der Körperoberfläche eines Patienten an eine die elektrischen Impulse verarbeitende Einrichtung, umfassend wenigstens:
einen äußeren Träger (1) und wenigstens einen inneren Träger (3, 3', 3"), wobei der äußeren Träger (1) und der wenigstens eine innere Träger (3, 3', 3") an einer definierten Anzahl von geometrisch verteilten Punkten (2) verbunden sind,

wobei die Punkte (2) einen äußeren Rand (4) des wenigstens einen inneren Trägers (3, 3', 3") bilden und perforiert sind, so dass der äußere Träger (1) und der wenigstens eine innere Träger (3, 3', 3") an diesen Punkten (2) trennbar sind,
einen an der Unterseite des äußeren Trägers (1) und des wenigstens einen inneren Trägers (3, 3', 3") ausgebildeten Haftbereich (5) zum Haften der medizinischen Elektrode auf der Körperoberfläche des Patienten und
ein Kontaktelement (7) und einen konzentrisch zu dem Kontaktelement (7) angeordneten leitfähigen Bereich (8) zur Herstellung von Kontakt mit der Körperoberfläche des Patienten, wobei das Kontaktelement (7) und der leitfähige Bereich (8) innerhalb des wenigstens einen inneren Trägers (3, 3', 3") angeordnet sind.

## Beschreibung

Die Erfindung betrifft eine medizinische Elektrode gemäß Anspruch 1.

Aus der Praxis sind medizinische Elektroden zur Erfassung und Übertragung von elektrischen Impulsen von der Körperoberfläche eines Patienten an eine die elektrischen Impulse verarbeitende Einrichtung bekannt.

Für unterschiedliche Anwendungen und entsprechend der Größe der Patienten sind medizinische Elektroden mit unterschiedlichen Dimensionen verfügbar. Beispielsweise kommen im neonatalen Bereich Elektroden mit einem Durchmesser von ca. fünfzehn Millimeter zur Anwendung, wohingegen Elektroden für Erwachsene einen Durchmesser von ca. fünfundzwanzig Millimeter bis sechzig Millimeter aufweisen können. Diese Vielzahl von medizinischen Elektroden unterschiedlicher Größe erweist sich in der medizinischen Praxis als nachteilig. So müssen beispielsweise medizinische Elektroden in wenigstens drei verschiedenen Maßen vorrätig gehalten werden, nämlich für neonatale Patienten und für Erwachsene mit kleiner, mittlerer oder großer Gestalt. Dies erfordert einen erhöhten logistischen Aufwand sowohl für den Hersteller und/oder Vertreiber der medizinischen Elektroden als auch für die Anwender in der medizinischen Praxis.

Die Aufgabe der vorliegenden Erfindung besteht darin, eine medizinische Elektrode anzugeben, mittels der die aufgezeigten Nachteile im Stand der Technik überwunden werden können.

Die erfindungsgemäße Aufgabe wird gelöst durch eine medizinische Elektrode mit den Merkmalen des Anspruchs 1.

So wird erfindungsgemäß eine medizinische Elektrode zur Erfassung und Übertragung von elektrischen Impulsen von der Körperoberfläche eines Patienten an eine die elektrischen Impulse verarbeitende Einrichtung vorgeschlagen, welche wenigstens einen äußeren Träger und wenigstens einen inneren Träger aufweist, wobei der äußeren Träger und der wenigstens eine innere Träger an einer definierten Anzahl von geometrisch verteilten Punkten verbunden sind und diese Punkte einen äußeren Rand des wenigstens einen inneren Trägers bilden und perforiert sind, so dass der äußere Träger und der wenigstens eine innere Träger an den Punkten trennbar sind. Ferner sind an der Unterseite des äußeren Trägers und des wenigstens einen inneren Trägers jeweils ein Haftbereich zum Haften der medizinischen Elektrode auf der Körperoberfläche des Patienten ausgebildet. Ein leitfähiger Bereich zur Herstellung von Kontakt mit der Körperoberfläche des Patienten ist konzentrisch zu einem Kontaktelement angeordnet, wobei das Kontaktelement und der leitfähige Bereich innerhalb des wenigstens einen inneren Trägers angeordnet sind.

Der Aufbau der erfindungsgemäßen medizinischen Elektrode ermöglicht in einfacher Weise vor einer Anwendung an einem Patienten die medizinischen Elektrode von einer größeren Form in eine kleinere Form zu ändern. Die Abmaße des äußeren Trägers entsprechen dabei der Elektrode mit der größeren Form und die Abmaße des wenigstens einen inneren Trägers der Elektrode der kleineren Form. Ein äußerer Rand der erfindungsgemäßen medizinischen Elektrode kann dabei vorzugsweise eine nahezu ovale Form aufweisen. In einer weiteren vorteilhaften Ausführungsform der erfindungsgemäßen medizinischen Elektrode kann der äußere Rand des äußeren Trägers eine rechteckige, vorzugsweise quadratische Form aufweisen.

Mit der erfindungsgemäßen medizinischen Elektrode können dem Anwender in vorteilhafter Weise wenigstens zwei medizinische Elektroden verschiedener Größe zur Verfügung gestellt werden, wobei von dem Anwender auf einfache und schnelle Weise die jeweilige erforderliche Elektrodengröße herstellbar ist.

Ein Vorteil ist zudem, dass in der klinischen Praxis mit der erfindungsgemäßen medizinischen Elektrode nur ein Produkt für eine Anwendung an wenigstens zwei Patienten unterschiedlicher Größe bevorratet werden muss.

Der innere Träger der erfindungsgemäßen medizinischen Elektrode ist in einer weiter bevorzugten Ausführungsform als ein erster und einen zweiter innerer Träger ausgebildet. Der erste und der zweite innere Träger sind in dieser Ausführung an einer definierten Anzahl von geometrisch verteilten Punkten verbunden, wobei die Punkte einen äußeren Rand des ersten inneren Trägers bilden und perforiert sind, so dass der erste innere Trägers und der zweite innere Träger an diesen Punkten trennbar sind. Mit dieser Ausführungsform können drei verschiedene Elektrodengrößen verfügbar gemacht werden.

In einer wiederum weiter bevorzugten Ausführungsform hat die erfindungsgemäße medizinische Elektrode einen Anfassbereich, der von einem Teil des äußeren Randes des wenigstens einen inneren Trägers begrenzt wird, wobei dieser Teil des äußeren Randes vorzugsweise durchgehend perforiert ist. Damit kann bei einer erforderlich kleineren Elektrodenabmessung auf einfache Weise der innere Träger von dem äußeren Träger der erfindungsgemäßen medizinischen Elektrode gelöst werden.

In einer Ausgestaltung der erfindungsgemäßen medizinischen Elektrode mit drei verschiedenen Elektrodengrößen, das heißt mit einer ersten Elektrodengröße entsprechend den Abmessungen des äußeren Trägers, einer zweiten Elektrodengröße entsprechend den Abmessungen des zweiten inneren Trägers und einer dritten Elektrodengröße entsprechend den Abmessungen des ersten inneren Trägers, ist in vorteilhafter Weise der Anfassbereich des ersten inneren Trägers und der Anfassbereich des zweiten inneren Trägers gegenüberliegend angeordnet. Diese Ausführungsform ermöglicht eine optimale Handhabung bei der Auswahl und Herstellung der entsprechenden Elektrodengröße vor deren Gebrauch.

Die vorliegende Erfindung wird mit Bezug auf die beigefügten Zeichnungen detaillierter erläutert, wobei gleiche Bezugszeichen gleiche Strukturen bezeichnen.

In der Zeichnung gilt:
- Figur 1: zeigt eine erfindungsgemäße medizinische Elektrode in einer Draufsicht,
- Figur 2: zeigt die erfindungsgemäße medizinische Elektrode der Fig. 1 in einer unteren Ansicht,
- Figur 3: zeigt eine Schnittdarstellung durch die vertikale Achse der erfindungsgemäßen medizinischen Elektrode der Fig. 1,
- Figur 4A: zeigt eine erfindungsgemäße medizinische Elektrode mit drei verschiedenen Elektrodengrößen mit zwei inneren Trägern und einem äußeren Träger,
- Figur 4B: zeigt die erfindungsgemäße medizinische Elektrode in der Größe der Abmaßen des äußeren Trägers,
- Figur 4C: zeigt die erfindungsgemäße medizinische Elektrode in der Größe der Abmaßen des zweiten inneren Trägers,
- Figur 4D: zeigt die erfindungsgemäße medizinische Elektrode in der Größe der Abmaßen des ersten inneren Trägers und
- Figur 5: zeigt eine Ausführung der erfindungsgemäßen medizinischen Elektrode mit einer quadratischen Bauform.

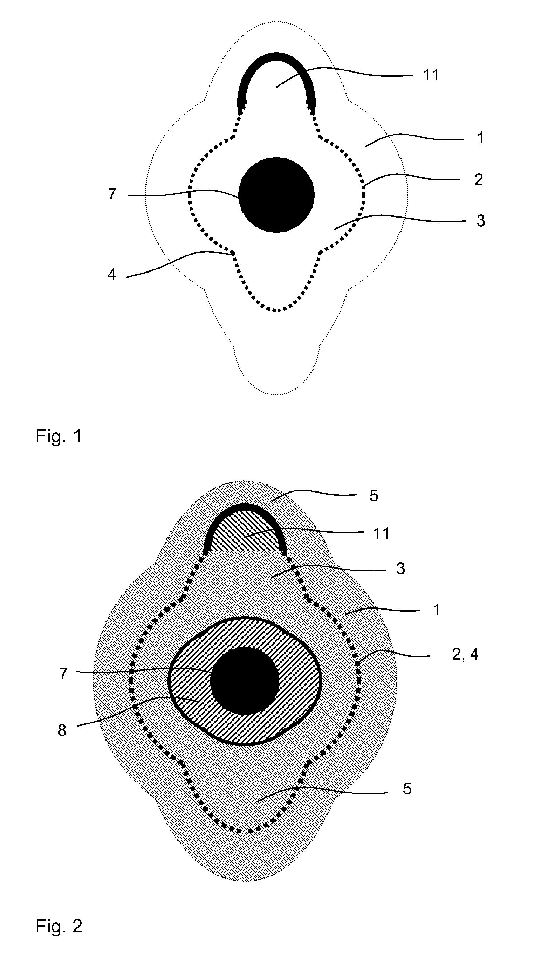

Wie in der Darstellung der Figur 1 zu erkennen ist, weist die erfindungsgemäße medizinische Elektrode einen äußeren Träger 1 und einen inneren Träger 3 auf.

Der äußeren Träger 1 und der innere Träger 3 sind an einer definierten Anzahl von geometrisch verteilten Punkten 2 verbunden. Die Punkte bilden einen äußeren Rand 4 des inneren Trägers 3 und sind perforiert. Die Punkte 2 sind derart perforiert, dass der innere Träger 3 und der äußeren Träger 1 trennbar sind. Somit kann die erfindungsgemäße medizinische Elektrode in einer entsprechenden Größe, gemäß der Größe des Patienten, schnell und einfach hergestellt werden. Der innere Träger 3 wird dazu von dem äußeren Träger 1 getrennt. Dabei sind in vorteilhafter Weise der innere Träger 3 mit einem Anfassbereich 11 ausgeführt, der von einem Teil des äußeren Randes 4 des inneren Trägers 3 begrenzt wird. Der Randbereich des Anfassbereiches 11, der mit einem Teil des äußeren Randes 4 des inneren Trägers 3 übereinstimmt, ist vorzugsweise durchgehend perforiert. Dies ermöglicht ein einfaches Trennen des inneren Trägers 3 von dem äußeren Träger 1. Der innere Träger 3 bildet zusammen mit dem äußeren Träger 1 die Grundfläche der erfindungsgemäßen medizinischen Elektrode in der größten Abmessung für die Anwendung für große Patienten. Für kleinere Patienten, wie bspw. Kinder, gelangt die erfindungsgemäße medizinische Elektrode mit der Grundfläche des inneren Trägers 3 zur Anwendung. Der Durchmesser des äußeren Trägers 1 kann im Bereich von fünfundfünfzig bis sechzig Millimeter liegen, der einer maximalen Elektrodengröße für Erwachsene entspricht. Vorzugsweise beträgt der Durchmesser des äußeren Trägers 1 sechzig Millimeter. Der innere Träger 3 besitzt vorzugsweise einen Durchmesser von dreißig Millimeter.

Das Trägermaterial besteht in vorteilhafter Weise aus einem geschäumten oder textilen Material, beispielsweise aus Pflastermaterial.

Der äußere Träger 1 ist in vorteilhafter Weise mit einer nahezu ovalen Form ausgebildet. Damit können Kollisionen der erfindungsgemäßen medizinischen Elektrode, zum Beispiel bei einer Brustwandableitung, vermieden werden.

Die erfindungsgemäße medizinische Elektrode ist in ihrem Zentrum mit einem Kontaktelement 7 ausgeführt, um das sich koaxial ein leitfähiger Bereich 8 (Figur 2) erstreckt. Das Kontaktelement 7 ist mit einem Kabel zur Verbindung mit einer die elektrischen Impulse verarbeitenden Einrichtung ausgeführt (nicht dargestellt). Das Kontaktelement 7 und der leitfähige Bereich 8 sind innerhalb des inneren Trägers 3 angeordnet. Das Kontaktelement 7 kann ein Material aus Stahl und Silberchlorid aufweisen. In einer besonders bevorzugten Ausführung ist das Kontaktelement 7 aus Kohlenstoff ausgeführt. Das Kabel zur Verbindung mit der die elektrischen Impulse verarbeitenden Einrichtung kann mit dem Kontaktelement 7 über eine Schweißverbindung verbunden sein.

Die elektrischen Impulse werden durch den in der Abbildung der Figur 2 dargestellten leitfähigen Bereich 8 von der Körperoberfläche des Patienten erfasst und über das Kontaktelement 7 und das Kabel an die die elektrischen Impulse verarbeitende Einrichtung weitergeleitet. Der leitfähige Bereich 8 ist mit einer Schicht Hydrogel ausgeführt. Die Abmaße sowohl des leitfähigen Bereiches 8 als auch des Kontaktelementes 7 sind für alle Endgrößen der erfindungsgemäße medizinische Elektrode gleich. Damit ist eine gute Signalqualität der elektrischen Impulse gewährleistet.

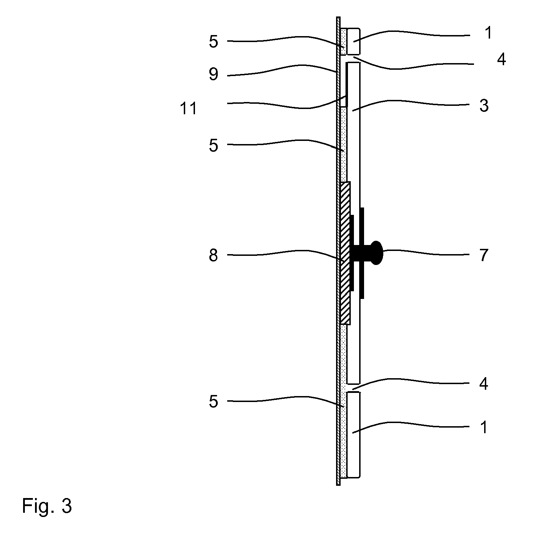

Die erfindungsgemäße medizinische Elektrode weist ferner an der Unterseite des äußeren Trägers 1 und des inneren Trägers 3 jeweils einen Haftbereich 5 auf (Figur 2), der ein Befestigen der Elektrode an die Körperoberfläche des Patienten ermöglicht. Mit Ausnahme eines Anfassbereiches 11 und dem leitfähigen Bereich 8 erstreckt sich der Haftbereich 5 vorzugsweise über die komplette untere Fläche der erfindungsgemäßen medizinischen Elektrode. Der Haftbereich 5 ist mit einer vor Gebrauch abzuziehenden Schutzabdeckung 9 (Figur 3) abgedeckt, wobei die Schutzabdeckung 9 vorzugsweise ohne Perforationen aufgebildet ist. Bei Gebrauch der erfindungsgemäßen medizinischen Elektrode und einer Auswahl einer Elektrodengröße, die der Grundfläche des inneren Trägers 3 entspricht, kann in vorteilhafter Weise in einem ersten Schritt die Schutzabdeckung 9 von der Unterseite der medizinischen Elektrode entfernt werden, bevor der innere Träger 3 mit dem Anfassbereich 11 von dem äußeren Träger 1 gelöst und in einem weiteren Schritt an der entsprechenden Position der Körperoberfläche des Patienten befestigt wird.

Zur Verdeutlichung des Aufbaus der erfindungsgemäßen medizinischen Elektrode der Fig. 1 ist in der Figur 3 eine Schnittdarstellung durch die vertikale Achse gezeigt.

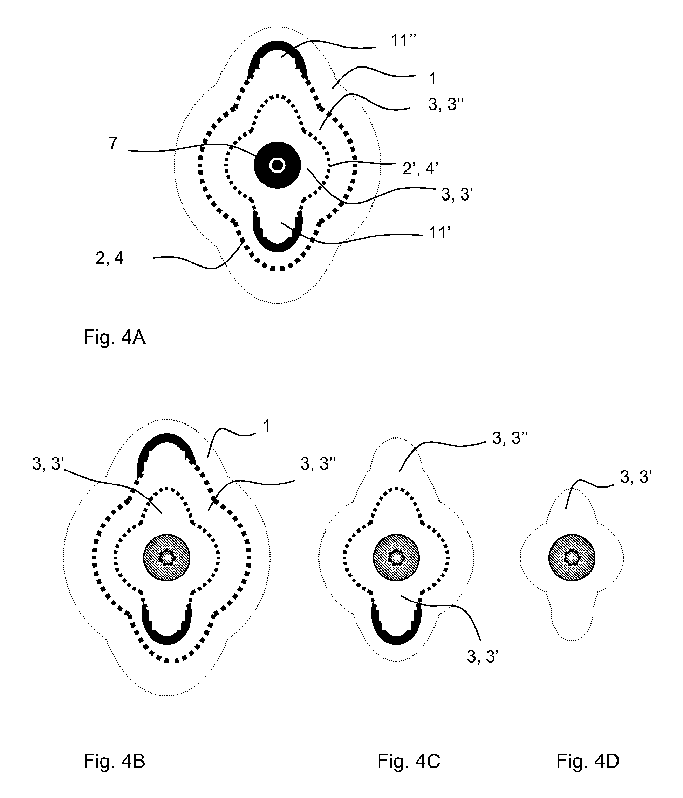

Die Darstellung der Figur 4A zeigt eine erfindungsgemäße medizinische Elektrode mit drei verschiedenen Elektrodengrößen; mit zwei inneren Trägern und einem äußeren Träger.

Gut zu erkennen ist, dass der innere Träger 3 als ein erster innerer Träger 3' und einen zweiter innerer Träger 3" ausgebildet ist, wobei der erster und der zweite innere Träger 3', 3" an einer definierten Anzahl von geometrisch verteilten Punkten 2' verbunden sind und die Punkte 2' einen äußeren Rand 4' des ersten inneren Trägers 3' bilden und perforiert sind. Der erste innere Träger 3' ist umgeben von dem zweite innere Träger 3" angeordnet, welcher wiederum von dem äußeren Träger 1 umgeben ist. Die Punkte 2' sind derart perforiert, dass der erste innere Träger 3' und zweite innere Träger 3" an diesen Punkten 2' trennbar sind.

Sowohl der erste innere Träger 3' als auch der zweite innere Träger 3" weisen jeweils einen Anfassbereich 11' und 11" auf, die in vorteilhafter Weise gegenüberliegend angeordnet sind. In dieser Ausgestaltung der erfindungsgemäßen medizinischen Elektrode ist ein leichtes Trennen sowohl des ersten inneren Trägers 3' von dem zweiten inneren Träger 3" als auch des ersten inneren Trägers 3' und des zweiten inneren Trägers 3" von dem äußeren Träger 1 ermöglicht. Der zweite inneren Träger 3" kann mit einem Durchmessers im Bereich von ca. fünfzehn bis zwanzig Millimeter im neonatalen Bereich zur Anwendung kommen. In der Darstellung der Figuren 4B bis 4D sind jeweils die drei unterschiedlich großen Elektroden gezeigt, die aus der erfindungsgemäße medizinische Elektrode der Darstellung der Figur 4A jeweils verfügbar gemacht werden können. Dabei ist in Figur 4B die erfindungsgemäße medizinische Elektrode in der Größe der Abmaße des äußeren Trägers 1 gezeigt. In dieser Ausführung sind der erste innere Träger 3', der zweite innere Träger 3" und der äußere Träger 1 verbunden. Die Figur 4C zeigt die erfindungsgemäße medizinische Elektrode in der Größe der Abmaße des zweiten inneren Trägers 3", in der der äußere Träger 1 von dem zweiten inneren Träger 3" getrennt ist. Die Figur 4D zeigt die erfindungsgemäße medizinische Elektrode in der Größe der Abmaße des ersten inneren Trägers 3', in der der erste innere Träger 3' von dem zweiten inneren Träger 3" und dem äußeren Träger 1 getrennt ist.

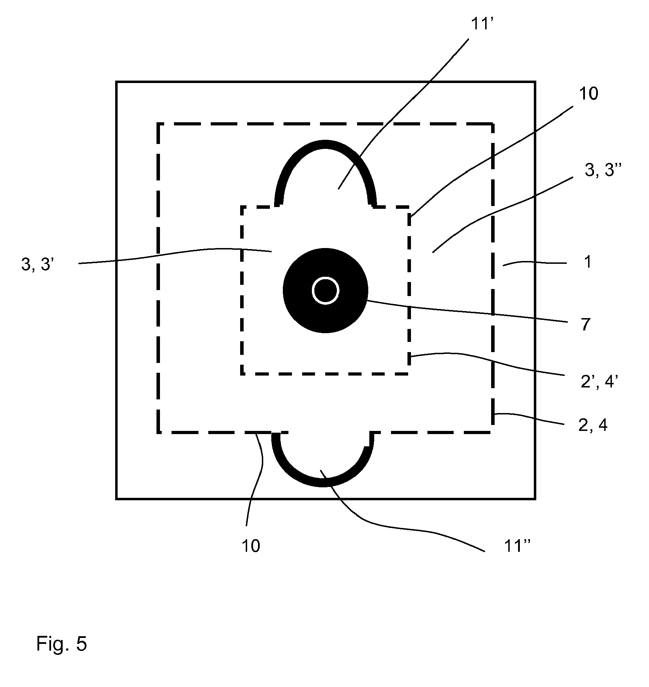

Figur 5 zeigt eine weitere mögliche Bauform der erfindungsgemäßen medizinischen Elektrode. Der äußere Träger 1, der erste innere Träger 3' und der zweite innere Träger 3" weisen eine quadratische Form auf. Die erfindungsgemäße medizinische Elektrode der Abbildung der Figur 5 ermöglicht in dieser Ausgestaltung drei verschiedene Größen. Die Punkte 2 bilden einen quadratischen äußeren Rand 4 des zweiten inneren Trägers 3". Die Punkte 2' bilden einen quadratischen äußeren Rand 4' des ersten inneren Trägers 3'.

Entsprechend der Darstellung der Figur 5 können die perforierten Punkte 2 und 2' in vorteilhafter Weise in Form von Langlöchern 10 ausgebildet sein. Der jeweilige Anfassbereich 11' und 11" des ersten inneren Trägers 3' und des zweiten inneren Trägers 3" sind wiederum in vorteilhafter Weise gegenüberliegend angeordnet. Mit dieser quadratischen Ausführung der erfindungsgemäßen medizinischen Elektrode lassen sich Verschnittverluste bei der Herstellung minimieren, wobei die Herstellkosten in vorteilhafter Weise reduziert werden können.

Die erfindungsgemäße Lösung stellt eine medizinische Elektrode sowohl für neonatale Patienten als auch erwachsene Patienten unterschiedlicher Größe bereit, welche auf einfache und schnelle Weise von dem Anwender in der jeweiligen Größe hergestellt werden kann. Dies ermöglicht eine kostengünstige Logistik und eine Verwendung der erfindungsgemäßen medizinischen Elektrode für verschiedene Anwendungen.

### BEZUGSZEICHENLISTE

- 1: Äußerer Träger
- 2, 2': Geometrisch verteilte Punkte
- 3: Innerer Träger
- 3': Erster innerer Träger
- 3": Zweiter innerer Träger
- 4: Äußerer Rand des inneren Trägers/ zweiten inneren Trägers
- 4': Äußerer Rand des ersten inneren Trägers
- 5: Haftbereich
- 7: Kontaktelement
- 8: Leitfähiger Bereich
- 9: Schutzabdeckung
- 10: Langlöcher
- 11: Anfassbereich des inneren Trägers
- 11': Anfassbereich des ersten inneren Trägers
- 11": Anfassbereich des zweiten inneren Trägers

## Patentansprüche

1. Medizinische Elektrode zur Erfassung und Übertragung von elektrischen Impulsen von der Körperoberfläche eines Patienten an eine die elektrischen Impulse verarbeitende Einrichtung, umfassend wenigstens:
einen äußeren Träger (1) und wenigstens einen inneren Träger (3, 3', 3"), wobei der äußeren Träger (1) und der wenigstens eine innere Träger (3, 3', 3") an einer definierten Anzahl von geometrisch verteilten Punkten (2) verbunden sind, wobei die Punkte (2) einen äußeren Rand (4) des wenigstens einen inneren Trägers (3, 3', 3") bilden und perforiert sind, so dass der äußere Träger (1) und der wenigstens eine innere Träger (3, 3', 3") an diesen Punkten (2) trennbar sind,
einen an der Unterseite des äußeren Trägers (1) und des wenigstens einen inneren Trägers (3, 3', 3") ausgebildeten Haftbereich (5) zum Haften der medizinischen Elektrode auf der Körperoberfläche des Patienten und
ein Kontaktelement (7) und einen konzentrisch zu dem Kontaktelement (7) angeordneten leitfähigen Bereich (8) zur Herstellung von Kontakt mit der Körperoberfläche des Patienten, wobei das Kontaktelement (7) und der leitfähige Bereich (8) innerhalb des wenigstens einen inneren Trägers (3, 3', 3") angeordnet sind.

2. Medizinische Elektrode nach Anspruch 1, **dadurch gekennzeichnet, dass** der äußere Träger (1) eine nahezu ovale Form aufweist.

3. Medizinische Elektrode nach Anspruch 1, **dadurch gekennzeichnet, dass** der äußere Träger (1) eine rechteckige, vorzugsweise quadratische Form aufweist.

4. Medizinische Elektrode nach einem der vorhergehenden Ansprüche, **dadurch gekennzeichnet, dass** der innere Träger (3) als ein erster und einen zweiter innerer Träger (3', 3") ausgebildet ist, wobei der erster und der zweite innere Träger (3', 3") an einer definierten Anzahl von geometrisch verteilten Punkten (2') verbunden sind, wobei die Punkte (2') einen äußeren Rand (4') des ersten inneren Trägers (3') bilden und perforiert sind, so dass der erste innere Träger (3') und zweite innere Träger (3") an diesen Punkten (2') trennbar sind.

5. Medizinische Elektrode nach einem der vorhergehenden Ansprüche, **dadurch gekennzeichnet, dass** an der Unterseite des äußeren Trägers (1) und des inneren Trägers (3, 3', 3") eine vor Gebrauch abzuziehende Schutzabdeckung (9) ausgebildet ist.

6. Medizinische Elektrode nach einem der vorhergehenden Ansprüche, **dadurch gekennzeichnet, dass** der äußere Träger (1) und der innere Träger (3, 3', 3") aus einem geschäumten oder textilen Material besteht.

7. Medizinische Elektrode nach einem der vorhergehenden Ansprüche, **dadurch gekennzeichnet, dass** der leitfähige Bereich (8) wenigstens aus einer Schicht Hydrogel besteht.

8. Medizinische Elektrode nach einem der vorhergehenden Ansprüche, **dadurch gekennzeichnet, dass** das Kontaktelement (7) mit einem Kabel zur Verbindung mit der die elektrischen Impulse verarbeitenden Einrichtung ausgeführt ist und vorzugsweise das Kabel und das Kontaktelement (7) über eine Schweißverbindung verbunden sind.

9. Medizinische Elektrode nach einem der vorhergehenden Ansprüche, **dadurch gekennzeichnet, dass** das Kontaktelement (7) aus Kohlenstoff ausgeführt ist.

10. Medizinische Elektrode nach einem der vorhergehenden Ansprüche, **dadurch gekennzeichnet, dass** die perforierten Punkte (2, 2') in Form von Langlöchern (10) ausgebildet sind.

11. Medizinische Elektrode nach einem der vorhergehenden Ansprüche, **dadurch gekennzeichnet, dass** der wenigstens eine innere Träger (3, 3', 3") einen Anfassbereich (11, 11', 11") aufweist, der von einem Teil des äußeren Randes (4, 4') des wenigstens einen inneren Trägers (3, 3', 3") begrenzt wird, wobei dieser Teil des äußeren Randes (4, 4') vorzugsweise durchgehend perforiert ist.

12. Medizinische Elektrode nach Anspruch 11, **dadurch gekennzeichnet, dass** der Haftbereich (5) im Anfassbereich (11, 11', 11 ") unterbrochen ist.

13. Medizinische Elektrode nach den Ansprüchen 4 und 11, **dadurch gekennzeichnet, dass** der Anfassbereich (11') des ersten inneren Trägers (3') und der Anfassbereich (11 ") des zweiten inneren Trägers (3") gegenüberliegend angeordnet sind.
